# EUROPÄISCHE PATENTSCHRIFT

(11) **EP 0 410 168 B1**
(45) Veröffentlichungstag und Bekanntmachung des Hinweises auf die Patenterteilung: **14.12.1994**
(21) Anmeldenummer: 90112742.3
(22) Anmeldetag: 04.07.1990
(51) Int. Cl.: C07C 273/18, C07C 275/06

(54) **Verfahren zur Herstellung unsymmetrisch substituierter Harnstoffe**
Process for the preparation of asymmetrically substituted ureas
Procédé de préparation d'urées substituées asymétriquement

(30) Priorität: 28.07.1989 AT 1830/89
(43) Veröffentlichungstag der Anmeldung: 30.01.1991
(73) Patentinhaber: Chemie Linz GmbH, A-4021 Linz (AT)
(72) Erfinder: Müllner, Martin, Dipl.-Ing.Dr., A-4050 Traun (AT); Stern, Gerhard, Dipl.-Ing.Dr., A-4180 Sonnberg (AT); Schulz, Erich, A-4052 Ansfelden (AT)
(74) Vertreter: Kunz, Ekkehard, Dr.

(56) Entgegenhaltungen:
- CHEMICAL REVIEWS, Bd. 72, Nr. 5, Oktober 1972, Easton, US, Seiten 457-496; S. OZAKI: "Recent advances in isocyanate chemistry"

## Beschreibung

Die vorliegende Erfindung betrifft ein Verfahren zur Herstellung unsymmetrisch substituierter Harnstoffe durch Umsetzung eines gasförmigen Gemisches einer Temperatur von 250 bis 600 °C aus Isocyansäure und Ammoniak mit eine primaren oder sekundären Amin.

Isocyansäure stellt einen wertvollen, reaktiven C-1 Baustein für die Synthese organischer Verbindungen dar. Unter anderem kann Isocyansäure durch Umsetzung mit primären oder sekundären Aminen zur Herstellung unsymmetrisch substituierter Harnstoffe verwendet werden.
Gemische aus Isocyansäure und Ammoniak dienen u.a. als Ausgangsmaterial für die Synthese von Melamin und werden durch thermische Zersetzung von Harnstoff, beispielsweise nach EP- A 124 704 erhalten. Die Isolierung der Isocyansäure aus diesem Gemisch bereitet jedoch Schwierigkeiten, da sich beim Abkühlen des Ammoniak-Isocyansäuregemisches Ammoniumisocyanat bildet, das sehr leicht wieder zu Harnstoff isomerisiert.

Es wurde nun unerwarteterweise gefunden, daß es zur Herstellung unsymmetrisch substituierter Harnstoffe aus Isocyansäure und primären oder sekundären Aminen gar nicht notwendig ist, die Isocyansäure aus dem gasförmigen Gemisch us Isocyansäure und Ammoniak zu isolieren, vielmehr reagiert die Isocyansäure trotz der Anwesenheit des Ammoniaks nicht zu Ammoniumisocyanat, sondern mit dem primären oder sekundären Amin zu einem unsymmetrisch substituierten Harnstoff, der von Ammoniak leicht abgetrennt werden kann.

Gegenstand der Erfindung ist daher ein Verfahren zur Herstellung unsymmetrisch substituierter Harnstoffe, das dadurch gekennzeichnet ist, daß ein gasförmiges Gemisch einer Temperatur von 250 bis 600 °C aus Isocyansäure und Ammoniak mit einem primaren oder sekundären Amin umgesetzt und der unsymmetrisch substituierte Harnstoff isoliert wird.

In einer Ausführungsform des erfindungsgemäßen Verfahrens, erfolgt die Reaktion in der Gasphase. Dabei wird ein primäres oder sekundäres Amin in ein gasförmiges Gemisch aus Isocyansaure und Ammoniak, das eine Temperatur von 250 bis 600 °C aufweist, eingebracht und anschließend das Reaktionsgemisch abgekuhlt, worauf der unsymmetrisch substituierte Harnstoff kondensiert und dann isoliert wird.
Das gasförmige Gemisch aus Isocyansäure und Ammoniak weist eine Temperatur von 250 bis 600 °C, bevorzugt von 300 bis 450 °C, besonders bevorzugt von 320 bis 380 °C, auf.

Unter primären oder sekundären Aminen sind solche Verbindungen zu verstehen, die eine oder mehrere Aminogruppen aufweisen. Sie können gegebenenfalls durch weitere unte den Reaktionsbedingungen inerte Gruppen substituiert sein. Beispiele hiefür sind gleich oder verschieden substituierte aliphatische, cycloaliphatische oder cyclische Amine , wie Methylamin, Ethylamin, Propylamin, Hexylamin, Dodecylamin, Hexadecylamin, Isopropylamin, Isobutylamin, Isooctylamin, tert. Butylamin, Methylethylamin, Ethylbutylamin, Cyclohexylamin oder Dimethylamin, Diethylamin, Diisopropylamin, Diisobutylamin, Pyrrolidin, Pyrrol, Piperidin, Morpholin, Ethylendiamin, Hexamethylendiamin, 4,4′-Diaminodicyclohexylmethan oder gleich oder verschieden substituierte aromatische Amine, wie Anilin, Nitroaniline, Chloraniline, Tolylamine, Benzylamin, Dibenzylamin, Naphthylamine, Phenylendiamine, Toluylendiamine, 4,4′-Diaminodiphenylmethan.

Das primäre oder sekundäre Amin kann in gasförmigem Zustand gegebenenfalls mit Hilfe eines Trägergases oder flüssig durch Einsprühen oder Eintropfen eingebracht werden, wobei das Amin in diesem Fall erst direkt in der Reaktionsmischung durch deren hohe Temperatur in den gasförmigen Zustand übergeführt wird. Das Amin kann dabei etwa äquimolar zu Isocyansäure zugegeben werden, oder es wird ein Überschuß des Amins eingesetzt. Bevorzugt werden pro Mol Isocyansäure 1 bis 7 Äquivalente, besonders bevorzugt 1,1 bis 3 Äquivalente Amin eingesetzt.
Das Gasgemisch kann dabei mit einem Trägergas verdünnt werden. Das Trägergas kann ein unter den Reaktionsbedingungen inertes Gas wie Stickstoff, Argon oder Ammoniak sein oder das gegebenenfalls gasförmig zugegebene primäre oder sekundäre Amin dient zusatzlich als Trägergas.
Die Kontaktzeit der Reaktanten ist dabei von der Größe der Apparatur und der Strömungsgeschwindigkeit der Gase abhängig, wobei im allgemeinen eine Kontaktzeit von einigen Sekunden ausreichend ist.

Das heiße, gasförmige Reaktionsgemisch wird anschließend gekühlt, wobei der unsymmetrisch substituiere Harnstoff als Niederschlag ausfällt. Es ist aber auch möglich, das heiße, gasförmige Reaktionsgemisch mit einem inerten Verdünnungsmittel durch Einleiten, über Füllkörperkolonnen, Wäscher u. dgl. in Kontakt zu bringen. Dabei kondensiert de gebildete Harnstoff in dem inerten Verdünnungsmittel.

In einer anderen Ausführungsform des Verfahrens wird das primare oder sekundäre Amin in flüssigem oder geschmolzenem Zustand oder verdünnt in einem unter den Reaktionsbedingungen inerten Verdünnungsmittel vorgelegt. Als primäres oder sekundäres Amin kommen die oben angeführten primären oder sekundären Amine in Frage. Das gasförmige Gemisch aus Isocyansäure und Ammoniak, das eine Temperatur von 250 bis 600 °C aufweist, wird in die Vorlage eingeleitet, wobei ein inertes Trägergas wie oben beschrieben benutzt werden kann. Das Amin kann auch selbst als Verdünnüngsmittel dienen und dadurch in einem hohen Überschuß vorliegen, oder es wird in einem unter den Reaktionsbedingungen inerten Verdünnungsmittel gelöst vorgelegt, wobei pro Mol Isocyansäure 1 bis 7 Mol, bevorzugt 1 bis 3 Mol Amin vorgelegt werden. Dabei hat sich unerwarteterweise herausgestellt, daß auch bei einem hohen Überschuß des Amins praktisch kein symmetrisch substituierter Harnstoff entsteht. Die Temperatur der Vorlage liegt unter dem Siedepunkt des Amins und des Verdunnüngsmittels, bevorzugt zwischen Raumtemperatur und 100 °C. Dabei kann gegebenenfalls auch eine Kühlung des flüssigen oder geschmolzenen oder gelösten Amin auf übliche Art und Weise, etwa mit Hilfe von Wärmeaustauschern, erfolgen. Wird ein geschmolzenes Amin vorgelegt, darf die Kühlung nicht dazu führen, daß das Amin wieder auskristallisiert.

Für beide Ausführungsformen des erfindungsgemäßen Verfahrens kommen als inerte Verdünnungsmittel Wasser oder organische Verdünnungsmittel, beispielsweise niedere aliphatische Alkohole, wie Methanol, Ethanol, Isopropanol, aliphatische Kohlenwasserstoffe, wie Pentan, Hexan, Heptan, aromatische Kohlenwasserstoffe, wie Benzol, Toluol, Xylol, halogenierte aliphatische Kohlenwasserstoffe, wie Methylenchlorid, Chloroform, Tetrachlorkohlenstoff, Ethylchlorid, Ethylenchlorid, halogenierte aromatische Kohlenwasserstoffe, wie Chlorbenzol, Trichlorbenzol, Ether, wie Diethylether, Diisopropylether, Dibutylether, Ethylmethylether, Dioxan, Carbonsäureamide, wie Dimethylformamid, N-Methylpyrrolidon oder Mischungen von oben angeführten Verdünnungsmitteln in Frage.

Nach beiden Ausführungsformen des erfindungsgemäßen Verfahrens liegt der gebildete Harnstoff danach je nach Natur des verwendeten Verdünnungsmittels bzw. je nach Natur des Harnstoffes und je nach der Art der Ausführungsform fest oder gelöst oder suspendiert in dem verwendeten inerten Verdünnungsmittel oder in dem verwendeten Amin vor.

Das bei der Reaktion gebildete Ammoniak entweicht und wird auf übliche Art und Weise abgeführt. Gegebenenfalls gelöste Ammoniakanteile können etwa mit Hilfe eines inerten Gases, wie beispielsweise Stickstoff oder Argon ausgetrieben werden.

Das Verfahren kann kontinuierlich oder diskontinuierlich durchgeführt werden, wird aber bevorzugt kontinuierlich durchgeführt.

Auf die oben beschriebene Art und Weise wird eine Lösung oder Suspension eines unsymmetrisch substituierten Harnstoffes erhalten, oder der unsymmetrisch substituierte Harnstoff fällt in fester Form an. Die Isolierung des gelösten oder festen Harnstoffes kann auf übliche Weise erfolgen, wie etwa durch Filtration, Abdampfen des Lösungsmittels der Herauslösen oder Abkratzen des Feststoffes, wobei gegebenenfalls eine Reinigung des Rückstandes durch übliche Methoden, wie Destillation, Umkristallisieren oder Chromatographie erfolgen kann.

Das Verfahren liefert unsymmetrisch substituierte Harnstoffe in guten Ausbeuten und guter Reinheit und stellt somit eine Bereicherung der Technik dar.

### Beispiel 1

125 g Harnstoff wurden pro Stunde kontinuierlich in einen Zersetzer eingetragen. Die Pyrolysegase wurden in einem beheizbaren Rohr bei 380°C mit 65 g Methylamin pro Stunde zur Reaktion gebracht.
Die Reaktionsgase wurden in einem Wäscher, der mit Wasser betrieben wurde, rasch auf Raumtemperatur abgekühlt.
Insgesamt wurden 750 g Harnstoff (12,5 Mol) und 389 g Methylamin (12,5 Mol) eingetragen.
Die Waschlösung wurde im Vakuum zur Trockene eingedampft und der Rückstand aus Ethanol umkristallisiert. Dabei wurden 426 g, das sind 46 % der Theorie, **Methylharnstoff** mit einem Schmelzpunkt von 100°C erhalten.

### Beispiel 2

Wie im Beispiel 1 beschrieben, aber unter Verwendung von 604 g Methylamin (19,4 Mol) wurde **Methylharnstoff** mit einem Schmelzpunkt von 100 °C in einer Ausbeute von 45 % der Theorie erhalten.

### Beispiel 3

Wie im Beispiel 1 beschrieben, aber unter Verwendung von 1164 g Methylamin (37,5 Mol) wurde **Methylharnstoff** mit einem Schmelzpunkt von 100 °C in einer Ausbeute von 45 % der Theorie erhalten.

### Beispiel 4

Wie im Beispiel 1 beschrieben, aber unter Verwendung von 250 g Harnstoff (4,2 Mol) und 273 g Propylamin (4,6 Mol) und unter Verwendung von N-Methylpyrrolidon als Verdünnungsmittel wurde nach dem Umkristallisieren aus Diisopropylether **Propylharnstoff** in einer Ausbeute von 37 % der Theorie erhalten.

| | | | | |
|---|---|---|---|---|
| C-H-N-Analyse: | theoret.: | C 46,7 %, | H 9,8 %, | N 27,2 % |
| | gef.: | C 46,4 %, | H 9,5 %, | N 27,3 % |

### Beispiel 5

Wie im Beispiel 4 beschrieben, aber unter Verwendung von 547 g Hexylamin (5,4 Mol) wurde nach dem Umkristallisieren aus Wasser **Hexylharnstoff** in einer Ausbeute von 74 % der Theorie erhalten.

| | | | | |
|---|---|---|---|---|
| C-H-N-Analyse: | theoret.: | C 58,3 %, | H 11,2 %, | N 19,4 % |
| | gef.: | C 58,5 %, | H 10,9 %, | N 19,3 % |

### Beispiel 6

250 g Harnstoff wurden innerhalb von 2 Stunden kontinuierlich in einen Zersetzer eingetragen. Die Pyrolysegase wurden in eine Schmelze von 1200 g Dodecylamin einer Temperatur von 80 bis 90 °C eingebracht. Nach beendeter Reaktion wu de das überschüssige Dodecylamin im Vakuum abdestilliert und der Rückstand aus Chloroform umkristallisiert. Dabei wurden 598,9 g, das sind 63 % der Theorie, **Dodecylharnstoff** mit einem Schmelzpunkt von 105 bis 107 °C erhalten.

### Beispiel 7

Wie im Beispiel 4 beschrieben, aber unter Verwendung von 459 g Ethylbutylamin (4,6 Mol) und Chlorbenzol als Verdünnungsmittel wurde nach Umkristallisieren aus n-Hexan **N,N-Ethylbutylharnstoff** in einer Ausbeute von 62 % de Theorie erhalten.
¹H-NMR: 0,89 (t,J=7,2 Hz, CH₃ (Butyl); 1,02 (t,J=7,0 Hz, CH₃ (Ethyl), 1,19-1,47(m, -CH₂-CH₂ (Butyl)); 3,08-3,20 (m, -CH₂-N-C=O); 5,78 (s, -NH₂).

### Beispiel 8

125 g Harnstoff wurden pro Stunde kontinuierlich in einen Zersetzer eingetragen. Die Pyrolysegase wurden in einem beheizbaren Rohr bei 380°C mit 120 g gasförmigem Dimethylamin pro Stunde zur Reaktion gebracht. Die Reaktionsgase wurden in einem Wäscher mit Wasser rasch auf Raumtemperatur abgekühlt.
Insgesamt wurden 146 g (2,4 Mol) Harnstoff und 160 g (3,6 Mol) Dimethylamin eingetragen.
Die Waschlösung wurde im Vakuum zur Trockene eingedampft und der Rückstand aus Ethanol umkristallisiert. Dabei wurden 117 g, das sind 55 % der Theorie, **N,N-Dimethylharnstoff** mit einem Schmelzpunkt von 178 - 184°C erhalten.

### Beispiel 9

Wie im Beispiel 4 beschrieben, aber unter Verwendung von 365,3 g Diethylamin (5,0 Mol) und unter Verwendung von n-Hexan als Verdünnungsmittel wurde nach dem Umkristallisieren aus Diisopropylether **N,N-Diethylharnstoff** mit einem Schmelzpunkt von 68 - 70 °C in einer Ausbeute von 30 % der Theorie erhalten.

### Beispiel 10

Wie im Beispiel 4 beschrieben, aber unter Verwendung von 368,9 g Isopropylamin (6,2 Mol) wurde nach dem Umkristallisieren aus Wasser **Isopropylharnstoff** mit einem Schmelzpunkt von 157 - 159 °C in einer Ausbeute von 39 % der Theorie erhalten.

### Beispiel 11

Wie im Beispiel 10 beschrieben, aber unter verwendung von Ethanol als Verdünnungsmittel wurde nach dem Umkristallisieren aus Wasser **Isopropylharnstoff** mit einem Schmelzpunkt von 157 - 159 °C in einer Ausbeute von 45 % der Theorie erhalten.

### Beispiel 12

Wie im Beispiel 4 beschrieben, aber unter Verwendung von 426,2 g Isobutylamin (5,8 Mol) und von Toluol als Verdünnungsmittel wurde nach dem Umkristallisieren aus Diisopropylether **Isobutylharnstoff** in einer Ausbeute von 70 % der Theorie erhalten
¹H-NMR: 0,84 (d,J=7,3 Hz, CH₃); 1,55-1,69 (m, CH); 2,80 (t,J=6,3 Hz, CH₂-N); 5,41 (s, -NH₂); 6,0 (s, breit, -NH-).

### Beispiel 13

Wie im Beispiel 12 beschrieben, aber unter Verwendung von 426,2 g tertiärem Butylamin (5,8 Mol) und N-Methyl-pyrrolidon als Lsöungsmittel wurde nach dem Umkristallisieren aus Wasser **tert. Butylharnstoff** mit einem Schmelzpunkt von 177 °C (Zers.) in einer Ausbeute von 55 % der Theorie erhalten.

### Beispiel 14

Wie im Beispiel 4 beschrieben, aber unter Verwendung von 391,2 g Cyclohexylamin (4,2 Mol) und Isopropanol als Verdünnungsmittel wurde nach dem Umkristallisieren aus Methanol **Cyclohexylharnstoff** mit einem Schmelzpunkt von 196 - 197 °C in einer Ausbeute von 48 % der Theorie erhalten.

### Beispiel 15

100 g Harnstoff wurden pro Stunde kontinuierlich in einen Zersetzer eingetragen. Die Pyrolysegase wurden in einem beheizbaren Rohr bei 320°C mit 250 g gasformigem Diisopropylamin pro Stunde zur Reaktion gebracht. Die Reaktionsgase wurden in einem Wäscher mit Chloroform rasch abgekühlt.
Insgesamt wurde 250 g (4,2 Mol) Harnstoff und 635 g (6,3 Mol) Diisopropylamin eingetragen. Dabei wurden nach dem Umkristallisieren aus Wasser 370 g **N,N-Diisopropylharnstoff,** das sind 60 % der Theorie, mit einem Schmelzpunkt von 98 - 101°C erhalten.

### Beispiel 16

Wie im Beispiel 4 beschrieben, aber unter Verwendung von 640,6 g Di-iso-butylamin (5,0 Mol) und N-Methyl-pyrrolidon als Verdünnungsmittel wurde nach dem Umkristallisieren aus n-Hexan **N,N-Diisobutylharnstoff** in einer Ausbeute von 30 % der Theorie, mit einem Schmelzpunkt von 73 - 75 °C erhalten.

### Beispiel 17

Wie im Beispiel 6 beschrieben, aber unte Vorlage von 1400 g Anilin einer Temperatur von 80 - 90 °C **Phenylharnstoff** mit einem Schmelzpunkt von 145 - 147 °C in einer Ausbeute von 41 % der Theorie erhalten.

### Beispiel 18

Wie im Beispiel 6 beschrieben, aber unter Vorlage von 1300 g Benzylamin einer Temperatur von 80 - 90 °C wurde nach dem Umkristallisieren aus Wasser **Benzylharnstoff** in einer Ausbeute von 71 % der Theorie erhalten.

| | | | | |
|---|---|---|---|---|
| C-H-N-Analyse: | theoret: | C 64,0 %, | H 6,7 %, | N 18,6 % |
| | gef.: | C 64,0 %, | H 6,7 %, | N 18,7 % |

### Beispiel 19

Wie im Beispiel 6 beschrieben, aber unter Vorlage einer 20%igen Lösung von Dibenzylamin in N-Methylpyrrolidon einer Temperatur von 70 °C wurde nach dem Umkristallisieren aus Wasser/Aceton **N,N-Dibenzylharnstoff** in einer Ausbeute von 62 % der Theorie erhalten.

| | | | | |
|---|---|---|---|---|
| C-H-N-Analyse: | theoret.: | C 75,0 %, | H 6,7 %, | N 11,7 % |
| | gef.: | C 74,8 %, | H 6,7 %, | N 11,6 % |

### Beispiel 20

Wie in Beispiel 6 beschrieben, aber unte Vorlage von einer 20%igen wäßrigen Lösung von Morpholin einer Temperatur von 80 °C wurde nach dem Umkristallisieren aus ethanol **Morpholin-4-carbonsäureamid** in einer Ausbeute von 71 % der Theorie erhalten.
¹H-NMR: 3,29 (t,J=4,7 Hz, N-CH₂); 3,57 (t,J=4,7 Hz, -O-CH₂); 6,16 (0=C-NH₂).
¹³C-NMR: 47,9 (-N-CH₂-); 70,0 (-O-CH₂-), 162,5 (C=O)

### Beispiel 21

Wie im Beispiel 20 beschrieben, aber unter Vorlage einer 20%igen ethanolischen Losung von Morpholin einer Temperatur von 25 °C wurde **Morpholin-4-carbonsäureamid** in einer Ausbeute von 53 % der Theorie erhalten.
¹H-NMR: 3,29 (t,J=4,7 Hz, N-CH₂); 3,57 (t,J=4,7 Hz, -O-CH₂); 6,16 (0=C-NH₂).
¹³C-NMR: 47,9 (-N-CH₂-); 70,0 (-O-CH₂-), 162,5 (C=O)

### Beispiel 22

Wie im Beispiel 6 beschrieben, aber unter Vorlage von 1300 g Morpholin einer Temperatur von 50 °C wurde nach dem Umkristallisieren aus Ethanol **Morpholin-4-carbonsäureamid** in einer Ausbeute von 53 % der Theorie erhalten.
¹H-NMR: 3,29 (t,J=4,7 Hz, N-CH₂); 3,57 (t,J=4,7 Hz, -O-CH₂); 6,16 (0=C-NH₂).
¹³C-NMR: 47,9 (-N-CH₂-); 70,0 (-O-CH₂-), 162,5 (C=O)

### Beispiel 23

Wie im Beispiel 6 beschrieben, aber unter Vorlage einer 20%igen Lösung von Pyrrolidin in Dioxan einer Temperatur von 25 °C wurde nach dem Umkristallisieren aus Wasser **Pyrrolidin-N-carbonsäureamid** in einer Ausbeute von 55 % der Theorie erhalten.

| | | | | |
|---|---|---|---|---|
| C-H-N-Analyse: | theoret.: | C 52,6 %, | H 8,8 %, | N 24,5 % |
| | gef.: | C 52,4 %, | H 8,7 %, | N 24,6 % |

### Beispiel 24

Wie im Beispiel 1 beschrieben, aber unter Verwendung von 300 g Ethylendiamin (5,0 Mol) wurde nach dem Umkristallisieren aus Wasser **Ethylendiharnstoff** in einer Ausbeute von 40 % der Theorie erhalten.

| | | | | |
|---|---|---|---|---|
| C-H-N-Analyse: | theoret.: | C 32,9 %, | H 6,9 %, | N 38,3 % |
| | gef.: | C 32,8 %, | H 6,9 %, | N 38,2 % |

## Patentansprüche

1. Verfahren zur Herstellung unsymmetrisch substituierter Harnstoffe, dadurch gekennzeichnet, daß ein gasförmiges Gemisch einer Temperatur von 250 bis 600 °C aus Isocyansäure und Ammoniak mit einem primären oder sekundaren Amin umgesetzt und der unsymmetrisch substituierte Harnstoff isoliert wird.

2. Verfahren nach Anspruch 1, dadurch gekennzeichnet, daß ein primäres oder sekundäres Amin in ein gasförmiges Gemisch einer Temperatur von 250 bis 600 °C aus Isocyansäure und Ammoniak eingebracht und anschließend aas gasförmige Reaktionsgemisch abgekühlt wird, worauf der unsymmetrisch substituierte Harnstoff kondensiert und isoliert wird.

3. Verfahren nach Anspruch 1 oder 2, dadurch gekennzeichnet, daß das primäre oder sekundäre Amin bei einer Temperatur von 320 bis 380°C eingebracht wird.

4. Verfahren nach einem der Ansprüche 1 bis 3, dadurch gekennzeichnet, daß pro Mol Isocyansäure 1 bis 7 Äquivalente primäres oder sekundäres Amin eingebracht werden.

5. Verfahren nach einem der Ansprüche 1 bis 4, dadurch gekennzeichnet, daß pro Mol Isocyansäure 1,1 bis 3 Äquivalente primäres oder sekundäres Amin eingebracht werden.

6. Verfahren nach einem der Ansprüche 1 bis 5, dadurch gekennzeichnet, daß das Reaktionsgemisch in einem inerten Verdünnungsmittel abgekühlt wird.

7. Verfahren nach Anspruch 1, dadurch gekennzeichnet, daß ein gasförmiges Gemisch einer Temperatur von 250 bis 600 °C aus Isocyansäure und Ammoniak in ein primäres oder sekundäres Amin, das in flüssigem oder geschmolzenem Zustand oder in einem unter den Reaktionsbedingungen inerten Verdünnungsmittel verdünnt vorgelegt wird, eingebracht wird, worauf der unsymmetrisch substituierte Harnstoff aus dem Reaktionsgemisch isoliert wird.

8. Verfahren nach Anspruch 7, dadurch gekennzeichnet, daß das primäre oder sekundäre Amin in einem unter den Reaktionsbedingungen inerten Verdunnungsmittel gelöst vorgelegt wird.

9. Verfahren nach einem der Ansprüche 1 bis 8, dadurch gekennzeichnet, daß als inertes Verdünnungsmittel ein organisches Verdünnungsmittel eingesetzt wird.

10. Verfahren nach einem der Ansprüche 1 bis 8, dadurch gekennzeichnet, daß als inertes Verdünnungsmittel Wasser eingesetzt wird.

## Claims

1. Process for the preparation of asymmetrically substituted ureas, characterized in that a gaseous mixture of isocyanic acid and ammonia at a temperature of 250 to 600°C is reacted with a primary or secondary amine and the asymmetrically substituted urea is isolated.

2. Process according to Claim 1, characterized in that a primary or secondary amine is introduced into a gaseous mixture of isocyanic acid and ammonia at a temperature of 250 to 600°C and the gaseous reaction mixture is then cooled, after which the asymmetrically substituted urea is condensed and isolated.

3. Process according to Claim 1 or 2, characterized in that the primary or secondary amine is introduced at a temperature of 320 to 380°C.

4. Process according to one of Claims 1 to 3, characterized in that 1 to 7 equivalents of primary or secondary amine are introduced per mol of isocyanic acid.

5. Process according to one of Claims 1 to 4, characterized in that 1.1 to 3 equivalents of primary or secondary amine are introduced per mol of isocyanic acid.

6. Process according to one of Claims 1 to 5, characterized in that the reaction mixture is cooled in an inert diluent.

7. Process according to Claim 1, characterized in that a gaseous mixture of isocyanic acid and ammonia at a temperature of 250 to 600°C is introduced into a primary or secondary amine which is introduced in liquid or molten state or diluted in a diluent which is inert under the reaction conditions, after which the asymmetrically substituted urea is isolated from the reaction mixture.

8. Process according to Claim 7, characterized in that the primary or secondary amine is introduced dissolved in a diluent which is inert under the reaction conditions.

9. Process according to one of Claims 1 to 8, characterized in that an organic diluent is employed as the inert diluent.

10. Process according to one of Claims 1 to 8, characterized in that water is employed as the inert diluent.

## Revendications

1. Procédé de préparation d'urées substituées asymétriquement, caractérisé en ce qu'on fait réagir un mélange gazeux d'acide isocyanique et d'ammoniac, à une température de 250 à 600°C, avec une amine primaire ou secondaire et on isole l'urée substituée asymétriquement.

2. Procédé selon la revendication 1, caractérisé en ce qu'on introduit une amine primaire ou secondaire dans un mélange gazeux d'acide isocyanique et d'ammoniac, à une température de 250 à 600°C, et ensuite, on refroidit le mélange réactionnel gazeux, après quoi on condense l'urée substituée asymétriquement et on l'isole.

3. Procédé selon la revendication 1 ou 2, caractérisé en ce qu'on introduit l'amine primaire ou secondaire à une température de 320 à 380°C.

4. Procédé selon l'une des revendications 1 à 3, caractérisé en ce qu'on introduit 1 à 7 équivalents d'amine primaire ou secondaire par mole d'acide isocyanique.

5. Procédé selon l'une des revendications 1 à 4, caractérisé en ce qu'on introduit 1,1 à 3 équivalents d'amine primaire ou secondaire par mole d'acide isocyanique.

6. Procédé selon l'une des revendications 1 à 5, caractérisé en ce qu'on refroidit le mélange réactionnel dans un diluant inerte.

7. Procédé selon la revendication 1, caractérisé en ce qu'on introduit un mélange gazeux d'acide isocyanique et d'ammoniac, à une température de 250 à 600°C, dans une amine primaire ou secondaire qui est à l'état liquide ou fondu, ou diluée dans un diluant inerte dans les conditions de réaction, et ensuite, on isole du mélange réactionnel l'urée substituée asymétriquement.

8. Procédé selon la revendication 7, caractérisé en ce que l'amine primaire ou secondaire se présente à l'état dilué dans un diluant inerte dans les conditions de reaction.

9. Procédé selon l'une des revendications 1 à 8, caractérisé en ce qu'on utilise, comme diluant inerte, un diluant organique.

10. Procédé selon l'une des revendications 1 à 8, caractérisé en ce qu'on utilise de l'eau comme diluant inerte.
